# FASCICULE DE BREVET EUROPEEN

(11) **EP 0 623 336 B1**
(45) Date de publication et mention de la délivrance du brevet: **25.09.1996**
(21) Numéro de dépôt: 94400760.8
(22) Date de dépôt: 07.04.1994
(51) Int. Cl.: A61K 7/06, A61K 7/48

(54) **Composition cosmétique sous forme de gel aqueux**
Kosmetische Zusammensetzung in Form eines wässrigen Gels
Cosmetic composition in the form of an aqueous gel

(30) Priorité: 03.05.1993 FR 9305238
(43) Date de publication de la demande: 09.11.1994
(73) Titulaire: L'OREAL, F-75008 Paris (FR)
(72) Inventeur: Afriat, Isabelle, F-75014 Paris (FR); Fodor, Pierre, F-92380 Garches (FR); Pouget, Françoise, F-94120 Fontenay Sous Bois (FR)
(74) Mandataire: Peuscet, Jacques

(56) Documents cités:
- EP-A- 0 332 501
- WO-A-92/19216
- WO-A-93/01797
- US-A- 4 863 725
- US-A- 5 073 372
- US-A- 5 204 104

## Description

La présente invention concerne une composition cosmétique sous forme de gel aqueux.

Les compositions cosmétiques pour le soin de la peau étaient jusqu'à présent généralement formulées sous forme de crème ou de lait contenant de l'huile comme élément lubrifiant, la présence d'huile évitant le déssèchement de la peau et conférant de la douceur à celle-ci. Cependant, ces formulations à base d'huile ont un toucher gras et sont souvent collantes, ce qui est désagréable et gênant pour l'utilisateur.

Pour éviter ces inconvénients, on a proposé de préparer des compositions cosmétiques sous forme de gel aqueux ne contenant ni corps gras ni huile. Mais les gels, pour pouvoir être utilisés en cosmétique, doivent présenter les mêmes qualités d'hydratation de la peau que les compositions à base d'huile ou de corps gras utilisées jusqu'à présent, et de plus, d'une part, avoir un toucher soyeux et non collant et, d'autre part, pouvoir être prélevés et étalés facilement. Par ailleurs, les gels doivent être stables et compatibles avec les différents additifs généralement utilisés dans les compositions cosmétiques, en particulier ceux qui sont des électrolytes. Enfin, ils doivent être transparents pour avoir une présentation attractive.

Dans US-A 4 863 725, on a décrit un gel aqueux hydratant ne contenant pas d'huile, préparé à l'aide d'un polyméthacrylate de glycéryle comme agent gélifiant et contenant un polyol tel que la glycérine et le polyéthylèneglycol. Mais ce gel ne présente pas toutes les qualités cosmétiques désirées, en particulier il n'a pas le toucher soyeux désiré.

Selon la présente invention, on a trouvé que l'on peut obtenir une composition cosmétique ayant les qualités désirées en utilisant comme agent gélifiant un mélange de trois polymères connus séparément pour leur activité gélifiante : un copolymère d'acide acrylique réticulé, un copolymère d'acide acrylique et de monomère vinylique portant des fonctions azotées et un polyéthylèneglycol ayant un poids moléculaire élevé, les effets de ces différents polymères se combinant pour donner, de façon inattendue, le résultat désiré.

La présente invention a pour objet une composition cosmétique sous forme de gel aqueux contenant un agent gélifiant et un milieu aqueux, caractérisée par le fait que l'agent gélifiant est constitué par un mélange de :
a) au moins un polymère d'acide acrylique réticulé, au moins partiellement neutralisé, ayant un poids moléculaire compris entre 700 000 et 4 500 000 environ,
b) au moins un copolymère d'acide acrylique et de monomère vinylique portant des fonctions amine, amidine et nitrile et obtenu par hydrolyse de polyacrylonitrile, au moins partiellement neutralisé, ayant un poids moléculaire compris entre 100 000 et 200 000 environ, et
c) au moins un polyéthylèneglycol ayant un poids moléculaire compris entre 10⁵ et 3 x 10⁷ environ.

Cette composition cosmétique est dépourvue d'huiles et de corps gras non miscibles à l'eau ; par cette expression, on entend que la composition contient au maximum 1 % d'huile ou de corps gras non miscibles à l'eau.

De préférence, la composition cosmétique contient :
- de 0,1 à 1 % du (des) polymère(s) défini(s) en a),
- de 0,1 à 1 % du (des) copolymères défini(s) en b) et
- de 0,01 à 1 % du (des) polyéthylèneglycol(s) défini(s) en c).

Les acides polyacryliques réticulés sont des agents gélifiants largement utilisés pour gélifier des solutions aqueuses. Ils sont dénommés "CARBOMERS" dans le dictionnaire CFTA et ils sont commercialisés sous la dénomination "CARBOPOL" par la société "GOODRICH BF". Lorsqu'ils sont utilisés seuls, ils produisent des gels collants, au toucher désagréable. De plus, les gels obtenus sont sensibles aux électrolytes et leur viscosité décroît fortement en milieu salin.

Les copolymères d'acide acrylique et de monomère vinylique comportant des fonctions amine, amidine et nitrile sont dénommés "acide acrylique/acrylonitrogène" dans le dictionnaire CFTA. Ils sont par exemple commercialisés sous la dénomination "HYPAN SA 100 H" par la société "LIPO CHEMICALS INC". Lorsqu'ils sont utilisés seuls, ils donnent des gels cassants, qui sont, par conséquent, difficiles à prélever, par exemple dans un pot, et difficiles à étaler sur la peau.

Les polyéthylèneglycols à poids moléculaire élevé sont des polyéthylèneglycols de formule

H(OCH₂CH₂)ₙOH

dans laquelle n est tel que le poids moléculaire soit supérieur à 10⁵ et inférieur à 3 x 10⁷. On utilise plus particulièrement un polyéthylèneglycol ayant un poids moléculaire voisin de 15 millions. On peut utiliser, en particulier, les polyéthylèneglycols commercialisés sous la dénomination commerciale "POLYOX" par la société "UNION CARBIDE". Ces polyéthylèneglycols forment des solutions épaissies qui, appliquées sur la peau, lui donnent un toucher doux et soyeux. Mais leur pouvoir épaississant est faible et, seuls, ils ne permettent pas d'obtenir un gel. De plus, il est connu qu'ils réagissent avec les acides polyacryliques pour former un précipité.

Selon la présente invention, on a trouvé qu'en combinant les trois polymères définis ci-dessus, on obtient un gel qui est moins sensible aux électrolytes que les polymères d'acide acrylique réticulés comme il sera montré ci-après. De plus, de façon surprenante, bien que des polyéthylèneglycols et des acides polyacryliques soient en contact, il ne se forme pas de précipité et, par conséquent, le gel reste transparent. Enfin, le gel obtenu selon l'invention peut être facilement prélevé et étalé sur la peau et il n'est pas collant.

Le polymère d'acide acrylique réticulé défini sous a) et le copolymère d'acide acrylique et de monomère vinylique défini sous b) doivent être au moins partiellement neutralisés pour pouvoir gonfler sous forme de gel. La neutralisation est effectuée à l'aide de toute base mais on utilise, de préférence, les hydroxydes alcalins, les alcanolamines par exemple la mono-, di- ou triéthanolamine, l'aminométhylpropanol, l'aminométhylpropanediol, le tris(hydroxyméthyl)aminométhane ou la N,N,N',N'-tétrakis-(2-hydroxypropyl)éthylènediamine.

La composition, selon la présente invention, peut éventuellement contenir un autre type de polymère comme agent gélifiant et hydratant, à savoir : un polyacrylate et/ou polyméthacrylate de glycéryle ayant avantageusement une viscosité de 400 à 5 000 pascals.secondes telle que mesurée à 20°C au viscosimètre BROOKFIELD RTV. Selon l'invention, on peut, par exemple, utiliser le polyméthacrylate de glycéryle commercialisé sous la dénomination "LUBRAJEL MS" par la société "GUARDIAN INC." ou le polyacrylate de glycéryle commercialisé sous la dénomination "HISPAGEL 100" par la société "HISPANO CHIMICA". Le polyacrylate et/ou le polyméthacrylate de glycéryle représente avantageusement 0,01 à 50 % en poids de la composition.

Les compositions selon l'invention peuvent avantageusement contenir au moins un actif cosmétique et/ou au moins un adjuvant de formulation cosmétique. Cet actif et/ou cet adjuvant peut être tout actif ou tout adjuvant cosmétique connu, à condition qu'il soit soluble dans l'eau et soit compatible avec le gel.

L'actif cosmétique hydrosoluble peut être, par exemple, choisi dans le groupe formé par les agents humectants, tels que les polyols, en particulier la glycérine, le propylèneglycol, le sorbitol et les polyéthylèneglycols de faible poids moléculaire, des agents de soins de la peau, tels que l'allantoïne, le panthénol et les hydrolysats de protéine, les astringents, tels que les alcools inférieurs en C₁ à C₄, les filtres solaires et les agents anti-radicaux libres.

Selon la présente invention, le gel peut également contenir comme actif une quantité d'une huile hydrosoluble telle que les glycérides d'acides gras en C₆-C₁₂ oxyéthylénés, comme le produit commercialisé sous la dénomination "SOFTIGEN 767" par la société "DYNAMIT-NOBEL", qui est un mélange de glycérides caprylique et caprique oxyéthylénés à 6 moles d'oxyde d'éthylène. Ces "huiles hydrosolubles" sont utilisées en des proportions ne dépassant pas 10 % en poids sans nuire à la transparence du gel.

Selon l'(les) actif(s) utilisé(s), le gel peut avoir des applications différentes. Il peut être, par exemple, un gel hydratant, un gel tonifiant, un gel après rasage, un gel après soleil ou un gel solaire.

L'adjuvant de formulation cosmétique hydrosoluble peut être, par exemple, choisi dans le groupe formé par les colorants, les parfums, les conservateurs et les agents de pH.

### EXEMPLE 1

On a préparé, à titre comparatif, deux gels aqueux : un gel G₁, selon l'invention, contenant trois polymères gélifiants et un gel G₂ ne faisant pas partie de l'invention.

| Matières premières | G₁ (en g) | G₂ (en g) |
|---|---|---|
| - Acide polyacrylique réticulé commercialisé sous la dénomination "CARBOPOL 940" par la société "GOODRICH BF" | 0,25 | 0,16 |
| - Copolymère d'acide acrylique et de monomère vinylique commercialisé sous la dénomination "HYPAN SA 100 H" par la société "LIPO CHEMICALS" | 0,25 | - |
| - Polyéthylèneglycol commercialisé sous la dénomination "POLYOX COAGULANT" par la société "UNION CARBIDE" | 0,1 | - |
| - N,N,N',N'-tétrakis(2-hydroxypropyl) éthylènediamine commercialisé sous la dénomination "QUADROL L" par la société "BASF" | 0,3 | - |
| - Triéthanolamine | 0,25 | 0,16 |
| - Eau distillée qsp | 100 | 100 |
| Viscosité (en pascals.secondes) | 1,33 | 1,90 |
| pH | 6,6 ± 0,1 | 6,8 ± 0,1 |

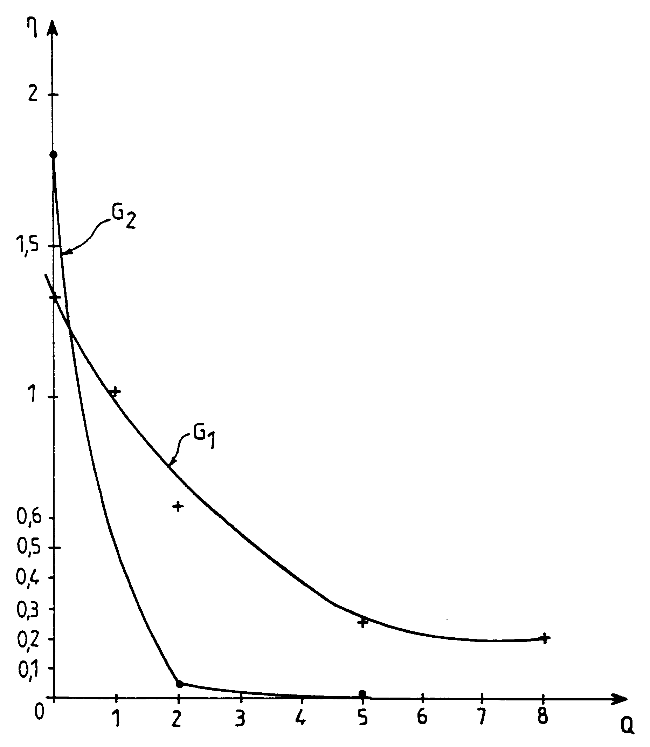

On a ajouté au gel obtenu des quantités croissantes d'une solution aqueuse de NaCl à 2 % (en poids) et on a mesuré la viscosité à 20° C à l'aide d'un viscosimètre CONTRAVES TV après chaque addition. Les résultats sont donnés sur les courbes de la figure 1 annexée où les quantités Q de solution de NaCl ajoutées sont indiquées en ml en abscisse et où la viscosité est donnée en pascals.secondes en ordonnée.

Ces courbes montrent que la viscosité du gel G₂ décroît beaucoup plus rapidement que celle du gel G₁ selon l'invention.

### EXEMPLE 2

On a préparé un gel hydratant pour la peau ayant la formulation suivante :

| | |
|---|---|
| - Acide polyacrylique réticulé commercialisé sous la dénomination "CARBOPOL 940" par la société " GOODRICH BF" | 0,28 g |
| - Copolymère d'acide acrylique et de monomère vinylique commercialisé sous la dénomination "HYPAN SA 100 H" par la société "LIPO CHEMICALS" | 0,15 g |
| - Polyéthylèneglycol de poids moléculaire 15 millions commercialisé sous la dénomination "POLYOX " par la société "UNION CARBIDE" | 0,10 g |
| - N,N,N',N'-tétrakis (2-hydroxypropyl) éthylènediamine commercialisé sous la dénomination "QUADROL L" par la société "BASF" | 0,21 g |
| - Triéthanolamine | 0,17 g |
| - Eau qsp | 100 g |

Le gel obtenu a une viscosité de 1,65 pascals.secondes et un pH de 5,8 ± 0,2. Il est stable, transparent, a un toucher soyeux et s'étale facilement.

### EXEMPLE 3

On a préparé un gel hydratant pour la peau ayant la formulation suivante :

| | |
|---|---|
| - Acide polyacrylique réticulé commercialisé sous la dénomination "CARBOPOL 940" par la société " GOODRICH BF" | 0,28 g |
| - Copolymère d'acide acrylique et de monomère vinylique commercialisé sous la dénomination "HYPAN SA 100 H" par la société "LIPO CHEMICALS" | 0,15 g |
| - Polyéthylèneglycol de poids moléculaire 15 millions commercialisé sous la dénomination "POLYOX " par la société "UNION CARBIDE" | 0,10 g |
| - Polyméthacrylate de glycéryle commercialisé sous la dénomination "LUBRAJEL MS" par la société "GUARDIAN INC" | 10 g |
| - N,N,N',N'-tétrakis (2-hydroxypropyl) éthylènediamine commercialisé sous la dénomination "QUADROL L" par la société "BASF" | 0,21 g |
| - Triéthanolamine | 0,17 g |
| - Eau qsp | 100 g |

Le gel obtenu a une viscosité de 1,7 pascals.secondes et un pH de 5,9 ± 0,2. Il est stable, transparent, a un toucher soyeux et s'étale facilement.

### EXEMPLE 4

On a préparé un gel hydratant pour la peau contenant des agents hydratants et ayant la formulation suivante :

| | |
|---|---|
| Acide polyacrylique réticulé commercialisé sous la dénomination "CARBOPOL 940" par la société " GOODRICH BF" | 0,28 g |
| - Copolymère d'acide acrylique et de monomère vinylique commercialisé sous la dénomination "HYPAN SA 100 H" par la société "LIPO CHEMICALS" | 0,15 g |
| - Polyéthylèneglycol de poids moléculaire 15 millions commercialisé sous la dénomination "POLYOX COAGULANT" par la société "UNION CARBIDE" | 0,10 g |
| - Polyméthacrylate de glycéryle commercialisé sous la dénomination "LUBRAJEL MS" par la société "GUARDIAN INC" | 20 g |
| - N,N,N',N'-tétrakis (2-hydroxypropyl) éthylènediamine commercialisé sous la dénomination "QUADROL L" par la société "BASF" | 0,21 g |
| - Triéthanolamine | 0,17 g |
| - Propylèneglycol | 3 g |
| - Glycérine | 3 g |
| - Polyéthylèneglycol comportant 20 unités oxyde d'éthylène commercialisé sous la dénomination "CARBOWAX 1000" par la société "GOODRICH BF" | 5 g |
| - Conservateurs qs | |
| - Eau qsp | 100 g |

Le gel obtenu a une viscosité de 2,65 pascals.secondes et un pH de 6,1 ± 0,2. Il est stable, transparent, a un toucher soyeux et s'étale facilement.

## Revendications

1. Composition cosmétique sous forme de gel aqueux contenant un agent gélifiant et un milieu aqueux, caractérisée par le fait que l'agent gélifiant est constitué par un mélange de :
a) au moins un polymère d'acide acrylique réticulé au moins partiellement neutralisé, ayant un poids moléculaire compris entre 700 000 et 4 500 000 environ,
b) au moins un copolymère d'acide acrylique et de monomère vinylique portant des fonctions amine, amidine et nitrile, et obtenu par hydrolyse de polyacrylonitrile, au moins partiellement neutralisé, ayant un poids moléculaire compris entre 100 000 et 200 000 environ, et
c) au moins un polyéthylèneglycol ayant un poids moléculaire compris entre 10⁵ et 3 x 10⁷ environ.

2. Composition cosmétique selon la revendication 1, caractérisée par le fait qu'elle contient :
- de 0,1 à 1 % en poids du (des) polymère(s) défini(s) en a),
- de 0,1 à 1 % en poids du (des) copolymère(s) défini(s) en b), et
- de 0,01 à 1 % en poids du (des) polyéthylèneglycol(s) défini(s) en c).

3. Composition cosmétique selon l'une des revendications 1 ou 2, caractérisée par le fait que le (les) polyéthylèneglycol(s) défini(s) en c) a (ont) un poids moléculaire voisin de 15 millions.

4. Composition cosmétique selon l'une des revendications 1 à 3, caractérisée par le fait que l'agent gélifiant contient un polyacrylate et/ou un polyméthacrylate de glycéryle.

5. Composition cosmétique selon la revendication 4, caractérisée par le fait que le poly(méth)acrylate de glycéryle a une viscosité de 400 à 5 000 pascals.secondes telle que mesurée à 20°C au viscosimètre BROOKFIELD RTV.

6. Composition cosmétique selon l'une des revendications 4 ou 5, caractérisée par le fait que le(s) poly(méth)acrylate(s) de glycéryle représente(nt) de 0,01 à 50 % en poids de la composition.

7. Composition cosmétique selon l'une des revendications 1 à 6, caractérisée par le fait qu'elle contient au moins un actif cosmétique hydrosoluble et/ou au moins un adjuvant de formulation.

8. Composition cosmétique selon la revendication 7, caractérisée par le fait que l'actif cosmétique hydrosoluble est choisi dans le groupe formé par les agents humectants, les agents de soins de la peau, les astringents, les filtres solaires et les agents anti-radicaux libres.

9. Composition cosmétique selon l'une des revendications 7 ou 8, caractérisée par le fait que l'actif cosmétique est une huile hydrosoluble en quantité ne dépassant pas 10 % en poids de la composition.

10. Composition cosmétique selon la revendication 7, caractérisée par le fait que l'adjuvant de formulation hydrosoluble est choisi dans le groupe formé par les colorants, les parfums, les conservateurs et les agents de pH.

## Claims

1. Cosmetic composition in the form of an aqueous gel containing a gelling agent and an aqueous medium, characterized in that the gelling agent consists of a mixture of:
a) at least one at least partially neutralized, cross-linked acrylic acid polymer having a molecular weight of between 700,000 and 4,500,000 approximately,
b) at least one at least partially neutralized copolymer of acrylic acid and vinyl monomer bearing amine, amidine and nitrile functions, and obtained by hydrolysis of polyacrylonitrile, having a molecular weight of between 100,000 and 200,000 approximately, and
c) at least one polyethylene glycol having a molecular weight of between 10⁵ and 3 × 10⁷ approximately.

2. Cosmetic composition according to Claim 1, characterized in that it contains:
- from 0.1 to 1% by weight of the polymer(s) defined in a),
- from 0.1 to 1% by weight of the copolymer(s) defined in b), and
- from 0.01 to 1% by weight of the polyethylene glycol(s) defined in c).

3. Cosmetic composition according to either of Claims 1 and 2, characterized in that the polyethylene glycol(s) defined in c) has/have a molecular weight in the region of 15 million.

4. Cosmetic composition according to one of Claims 1 to 3, characterized in that the gelling agent contains a poly(glyceryl acrylate) and/or poly(glyceryl methacrylate).

5. Cosmetic composition according to Claim 4, characterized in that the poly[glyceryl (meth)acrylate] has a viscosity of 400 to 5,000 pascal seconds as measured at 20°C using a BROOKFIELD RTV viscometer.

6. Cosmetic composition according to either of Claims 4 and 5, characterized in that the poly[glyceryl (meth)acrylate](s) represent(s) from 0.01 to 50% by weight of the composition.

7. Cosmetic composition according to one of Claims 1 to 6, characterized in that it contains at least one water-soluble cosmetic active agent and/or at least one formulation adjuvant.

8. Cosmetic composition according to Claim 7, characterized in that the water-soluble cosmetic active agent is chosen from the group composed of humectant agents, skin care agents, astringents, sunscreen agents and anti-free-radical agents.

9. Cosmetic composition according to either of Claims 7 and 8, characterized in that the cosmetic active agent is a water-soluble oil in a quantity not exceeding 10% by weight of the composition.

10. Cosmetic composition according to Claim 7, characterized in that the water-soluble formulation adjuvant is chosen from the group composed of colorants, perfumes, preservatives and pH agents.

## Patentansprüche

1. Kosmetisches Mittel in Form eines wäßrigen Gels, enthaltend ein gelbildendes Mittel und ein wäßriges Milieu, dadurch gekennzeichnet, daß das gelbildende Mittel aus einem Gemisch von:
a) wenigstens einem zumindest partiell neutralisierten Polymer aus vernetzter Acrylsäure mit einem Molekulargewicht von ungefähr 700 000 bis 4 500 000,
b) wenigstens einem zumindest partiell neutralisierten Copolymer aus Acrylsäure und Vinylmonomer, welches Amin-, Amidin- und Nitrilfunktionen trägt und durch Hydrolyse von Polyacrylnitril erhalten wurde, mit einem Molekulargewicht von ungefähr 100 000 bis 200 000 und
c) wenigstens einem Polyethylenglykol mit einem Molekulargewicht von ungefähr 10⁵ bis 3 x 10⁷
besteht.

2. Kosmetisches Mittel nach Anspruch 1, dadurch gekennzeichnet, daß es:
- 0,1 bis 1 Gew.-% des (der) unter a) definierten Polymers(e),
- 0,1 bis 1 Gew.-% des (der) unter b) definierten Copolymers(e) und
- 0,01 bis 1 Gew.-% des (der) unter c) definierten Polyethylenglykols(e)
enthält.

3. Kosmetisches Mittel nach einem der Ansprüche 1 oder 2, dadurch gekenzeichnet, daß das (die) unter c) definierte(n) Polyethylenglykol(e) ein Molekulargewicht von ungefähr 15 Millionen besitzt(en).

4. Kosmetisches Mittel nach einem der Ansprüche 1 bis 3, dadurch gekennzeichnet, daß das gelbildende Mittel ein Glycerin-Polyacrylat und/oder ein -Polymethacrylat enthält.

5. Kosmetisches Mittel nach Anspruch 4, dadurch gekennzeichnet, daß das Glycerin-Poly(meth)acrylat eine Viskosität von 400 bis 5 000 pa.s besitzt, gemessen bei 20°C mit dem BROOKFIELD RTV-Viskosimeter.

6. Kosmetisches Mittel nach einem der Ansprüche 4 oder 5, dadurch gekennzeichnet, daß das (die) Glycerin-Poly(meth)acrylat(e) 0,01 bis 50 Gew.-% des Mittels ausmacht(en).

7. Kosmetisches Mittel nach einem der Ansprüche 1 bis 6, dadurch gekennzeichnet, daß es wenigstens einen wasserlöslichen kosmetischen Wirkstoff und/oder wenigstens ein Formulierungsadjuvans enthält.

8. Kosmetisches Mittel nach Anspruch 7, dadurch gekennzeichnet, daß der wasserlösliche kosmetische Wirkstoff ausgewählt ist unter Feuchthaltemitteln, Hautpflegemitteln, adstringierenden Mitteln, Sonnenfiltern und Mitteln gegen freie Radikale.

9. Kosmetisches Mittel nach einem der Ansprüche 7 oder 8, dadurch gekennzeichnet, daß der kosmetische Wirkstoff ein wasserlösliches Öl ist, dessen Menge 10 Gew.-% des Mittels nicht übersteigt.

10. Kosmetisches Mittel nach Anspruch 7, dadurch gekennzeichnet, daß das wasserlösliche Formulierungsadjuvans ausgewählt ist unter Färbemitteln, Parfums, Konservierungsmitteln und pH-Mitteln.
